# EUROPEAN PATENT APPLICATION

(11) **EP 4 122 452 A1**
(43) Date of publication of application: **25.01.2023**
(21) Application number: 21187419.3
(22) Date of filing: 23.07.2021
(51) Int. Cl.: A61K 9/16, A61K 31/00, A61P 1/00

(54) **PHARMACEUTICAL COMPOSITION COMPRISING NILOTINIB AND METHOD OF MANUFACTURING THE SAME**

(71) Applicant: KRKA, d.d., Novo mesto, 8501 Novo mesto (SI)
(72) Inventor: BENKOVIC, Marko, 8000 Novo mesto (SI); PISANEC, Gasper, 8000 Novo mesto (SI); BIRSA CELIC, Tadeja, 8000 Novo mesto (SI); BARIC, Miha, 8000 Novo mesto (SI); KORASA, Klemen, 8000 Novo mesto (SI)
(74) Representative: Hoffmann Eitle

(57) **Abstract**

The present invention provides methods for preparing nilotinib-containing pharmaceutical compositions. The methods of the invention rely on a step of hot melt granulation employing a polyethylene glycol meltable binder. Pharmaceutical compositions obtainable by such methods are also provided.

## Description

### Technical field of the invention

The present invention concerns the technical field of pharmaceuticals. More specifically, the present invention provides pharmaceutical compositions comprising nilotinib and methods for making the same.

### Background of the invention

Nilotinib is a protein kinase inhibitor suitable, specifically a tyrosine kinase inhibitor, for the treatment of tumor diseases and especially Philadelphia-positive chronic myelogous leukaemia. It has the following chemical structure:

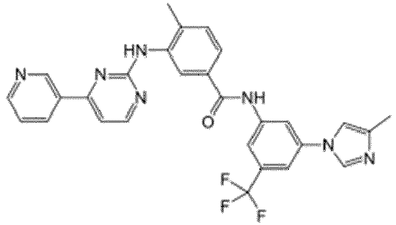

Several polymorphic forms of nilotinib are described in WO 2007/015870 A. Further polymorphic forms T1 to T19 are described in WO 2010/054056 A. Nilotinib is commercially available under the tradename Tasigna^{®} from Novartis in the form of a wet-granulated pharmaceutical composition in hard capsules having strengths of 50 mg, 150 mg or 200 mg. Alternative formulations are described *inter alia* in WO 2008/037716 A and EP 3 501 505 A. This latter document also provides a useful summary of further literature on nilotinib.

There is nonetheless a need for providing further pharmaceutical compositions comprising nilotinib as the active pharmaceutical ingredient. The present invention concerns nilotinib in polymorphic form A. This polymorphic form has poor flow characteristics, which may lead to handling problems. For instance, WO 2008/037716 A describes wet granulation of nilotinib and mentions sticking problems during automatic capsule filling in its Example 1. The present invention therefore has the objective of providing pharmaceutical compositions comprising nilotinib in form A, which exhibit good flow properties without compromising stability and/or solubility.

### Summary of the invention

The above objectives are accomplished by the methods and pharmaceutical compositions of the present invention, which rely on hot melt granulation as the production method and on the use of a polyethylene glycol (macrogol) as a meltable binder.

More specifically, the present invention relates to the methods and pharmaceutical compositions as specified in the appended claims. Further embodiments are derivable from the subsequent detailed description of the invention.

### Detailed description of the invention

### Definitions

A **meltable binder** in the sense of the present invention is a binder having a melting point in the range of from 30°C to 100°C and preferably 40°C to 65C.

In the context of the present invention, the term **binder** is used in the context of the present invention to characterize a substance capable of increasing adhesion between other particles contained in the pharmaceutical composition of the present invention.

In the context of the present invention, the **melting point** of a substance is the temperature at which the melted substance solidifies. This temperature is to be determined by melting 1g of a sample of the substance in a glass vessel and then allowing the substance to cool spontaneously, i.e. without taking any active cooling measures. The melting point is the temperature at which solidification begins to occur by visual inspection. When using a mixture of different meltable binders, the melting point of the meltable binder with the highest melting point is to be taken as the relevant melting point for determining the temperature of high shear mixing.

In the context of the present invention, the term **organic liquid** is used to characterize a substance that is in the liquid state under ambient conditions (1013,25 hPa and 22 °C).

In the context of the present invention, the term **high shear mixing** has the generally accepted meaning as described for instance in the Wikipedia entry "High-shear mixer" (https://en.wikipedia.org/wiki/High-shear mixer; version of July 22, 2021) and it refers in particular to any type of mixing which causes shearing that is at least as high as that accomplished when running the UltimaGral 25 L by GEA^{®} high shear mixer at an impeller speed of 50 rpm and the chopper at a speed of 1000 rpm.

In the context of the present invention, indications of **nilotinib amounts** refer to the amount of the free base, i.e. without taking weight contributions by salt components, hydrate or solvate components into account.

In the context of the present invention, **room temperature** is intended to refer to a temperature of 22°C.

### Overview

The present invention provides pharmaceutical compositions comprising nilotinib. The pharmaceutical compositions of the present invention are obtained by hot melt granulation optionally followed by mixing with extragranular further components.

A meltable binder is used in the granulation. The meltable binder is selected from polyethylene glycol binders and especially polyethylene glycol having a molecular weight of about 8000. The method and product of the present invention neither involve nor require the use of water or any other solvent.

The hot-melt granulation process of the present invention, in combination with the use of a polyethylene glycol meltable binder is suitable for providing a pharmaceutical composition comprising nilotinib, in which the active ingredient remains stable during manufacture and in the final product, and which allows to significantly improve the flow properties of the material.

### Nilotinib

As noted above, nilotinib can exist in multiple polymorphic forms. The present invention relies on the use of polymorphic form A, as described in WO 2007/015870 A, and in particular in anyone of paragraphs [0037], [0038], [0042] to [0044] and claims 1 to 3 and 7 to 18 of this document. According to a preferred embodiment, nilotinib is used in the form of the hydrochloride salt. This is also used in polymorphic form A. More preferably, it is characterized by an x-ray powder diffraction pattern having at least one, more preferably at least two, still more preferably at least four and most preferably all, maxima selected from about 8.5°, 11.0°, 11.5°, 17.2°, 18.8°, 19.2°, 20.8°, 22.1°, and 26.0° (2θ degrees), as described in paragraph [0043] of WO 2007/015870 A.

The presence of minor amounts of other solid forms of nilotinib in the starting material and/or in the final product is neither preferred not completely excluded. Preferably, other solid forms of nilotinib are present in relative amounts of 0 to 5 wt.%, preferably 0 to 1 wt.% and most preferably 0 wt.% with respect to the total weight of the nilotinib used for/contained in the pharmaceutical composition of the present invention

### Meltable binder

According to the present invention, polyethylene glycol (PEG) is used as the meltable binder. In the field of pharmaceuticals, PEG is frequently referred to as macrogol. Any pharmaceutical grade PEG may be used provided it shows a melting point in the range of from 30°C to 100°C and preferably in the range of from 40°C to 70°C. More preferably, the PEG has a molecular weight of 5000 to 12,000 g/mol, such as 6000 to 10,000 g/mol, in particular 7000 to 9000 g/mol and especially about 8000 g/mol is used. The molecular weight distribution may be monodisperse or polydisperse. The most preferred meltable binder is commercially available as Macrogol 8000, PEG 8000 or Carbowax 8000.

Optionally, one or more additional meltable binders may be present. It is advisable to select such optional further meltable binders such that said additional meltable binders do not interfere with the accomplishment of the beneficial effects of the present invention. It is however preferred that such further meltable binders are either completely absent or constitute only a minor fraction of 10 wt.% or less, more preferably 5 wt.% or less of the total weight of meltable binder contained in the pharmaceutical composition of the present invention.

### Optional Excipients

The pharmaceutical composition of the present invention may optionally contain one or more further excipients in addition to the polyethylene meltable binder. These may for instance be one or more of fillers, disintegrants, glidants, lubricants, surfactants

Preferred fillers include lactose or its derivatives, microcrystalline cellulose, mannitol, starch, powdered cellulose, sorbitol, compressible sugar and sucrose.

Preferred disintegrants include crospovidone, starch, pregelatinised starch, cross-linked carboxymethylcellulose.

Preferred glidants include colloidal silicon dioxide, talc, magnesium trisilicate.

Preferred lubricants include sodium stearyl fumarate, talc, magnesium stearate, calcium stearate, magnesium carbonate, polyethylene glycol glyceryl behenate, stearic acid, hydrogenated castor oil and glyceryl monostearate.

Preferred surfactants include Polysorbate 20, other types of polysorbates, Alkyl sulfates, alkyl aryl sulfonates and polyethylene glycols.

### Optional presence of excipients in extragranular phase

The pharmaceutical composition may optionally comprise extragranular components. That is, one or more of the above-mentioned excipients or any further components may be provided as extragranular components by incorporating them into the composition after the granulation, i.e. by mixing of the respective component with the granulate. The extragranular components are not particularly restricted and may include in particular one or more of fillers, glidants, lubricants, disintegrants and surfactants. These extragranular components may be independently selected from the corresponding lists provided above for the intragranular components, i.e. an extragranular disintegrant may be the same or different from the intragranular disintegrant.

In an embodiment, part of the filler is provided in the intragranular phase (i.e. incorporated before granulation) and a further part of the filler is provided in the extragranular phase (i.e. incorporated after granulation). It is further preferred to provide the entire lubricant and glidant components as extragranular components.

### Relative amounts

The pharmaceutical composition typically comprises nilotinib, meltable binder, and optional further excipients in the following relative amounts:
Nilotinib: 30 to 70 wt.%, preferably 40 to 60 wt.% and more preferably 45 to 55 wt.% (wherein the nilotinib weight is expressed as the weight of the free base, i.e. without taking into account any weight contributions by salt counterions, hydrates or solvates);
Meltable binder: 3 to 30 wt.%, preferably 5 to 25 wt.% and more preferably 8 to 20 wt.%;
Filler: 0 to 50 wt.%, preferably 10 to 40 wt.% and more preferably 15 to 35 wt.%;
Disintegrant: 0 to 10 wt.%, preferably 1 to 8 wt.% and more preferably 2 to 5 wt.%;
Glidant: 0 to 2 wt.%, preferably 0.1 to 1 wt.% and more preferably 0.2 to 0.8 wt.%;
Lubricant: 0 to 5 wt.%, preferably 1 to 3 wt.% and more preferably 1.5 to 2.5 wt.%;

The above relative amount indications are based on the total weight of the pharmaceutical composition (excluding the weight of a tablet film coating, a capsule shell or any other container) and they include/take into account the relative amounts of any components contained in the intragranular phase and the extragranular phase.

### Particle sizes, absence of water or other solvents

The particle sizes of the starting materials used in the present invention are not particularly restricted. Likewise, there is no particular restriction of the breadth of the particle size distribution.

The entire process of the invention is performed in the dry state, i.e. in the absence of solvents like water or organic solvents, and also in the absence of any other substance that is liquid at room temperature (e.g. for forming a dispersion).

### Hot melt granulation

Hot melt granulation is accomplished by a procedure comprising the steps of premixing to form the intragranular composition, high shear mixing of this composition to obtain a granulate and sieving of the granulate. These steps are described in more detail below.

### Premixing

The components of the intragranular composition, nilotinib, meltable binder and optional further excipients, are mixed as powders in the absence of a solvent to thereby yield the intragranular composition. Suitable equipment for this step includes, but is not limited to container mixers, tumbling mixers, ribbon mixers, planetary mixers and the like.

The powder blend is subsequently transferred to a high shear mixer.

### High shear mixing

The components are heated during mixing in a high shear mixer. The temperature of the high shear mixer heating coat is set to a temperature of 10-50 °C, preferably 15-35°C, above the melting point of the meltable binder. For instance, in case of macrogol 8000, which has a melting point of about 55°C, a temperature of the heating coat of 70-90°C is advantageous.

The equipment to be used for high shear mixing is not particularly limited. Typically, it includes an impeller and a chopper. A suitable high shear mixer is for example the UltimaGral 25 L by GEA^{®} or VG 65/10 by Glatt^{®} or GMA 70 by L.B. Bohle^{®}.

The components in the mixer are typically heated until the product reaches the melting point of the meltable binder and preferably a temperature of at least 10°C, for example a temperature of 10-15°C, above the melting point of the meltable binder. For instance, when using macrogol 8000, the temperature should preferably reach at least 65 °C. This leads to melting of the binder, such as Macrogol 8000, which allows granules to form. The heating (period of temperature increase) of the granulate normally lasts from 2 to 40 minutes and more typically from 5 to 30 min. During this heating (temperature increase) period, high shear mixing is performed, preferably under the following conditions: The high shear impeller is set at 50 to 300 rpm and the chopper is set at 1000 to 3000 rpm, the high shear impeller being turned on for 2-10 seconds in each minute while the chopper may be turned on or off as desired and for any duration, including, for example, being turned on for 2-10 seconds in each minute. Preferably, high shear impeller is set at 100 rpm and the chopper is set at 1500 rpm and both are turned on for 5 seconds in each minute.

The high shear mixing may optionally be continued after the product has reached the target temperature using preferably the same settings for the high shear mixer for a period between 0 to 10 min. Preferably, however, cooling is started immediately once the product has reached the target temperature.

At the end of the above heating period, the mass may be cooled while being mixed under high shear conditions until the meltable binder solidifies. In the case of Macrogol 8000, cooling is preferably performed until a temperature of 45°C or lower is reached. Subsequently, further cooling to a temperature between 20°C and 30 °C is carried out, preferably in a fluid bed dryer.

Alternatively, the mass may be transferred in a hot state to a fluid bed dryer and then be cooled in this device to a temperature between 20°C and 30°C.

### Sieving

After it is cooled, the granulate may be sieved through a sieve. For instance, the sieve may have openings in a size range of from 0.2 to 3.0 mm, preferably 0.4 to 2.5 mm and even more preferably from 0.5 to 2.2 mm, such as between 0.6 and 2.0 mm.

### Mixing with extragranular components

The mixing of the granulate with the extragranular components (if any) yields the final pharmaceutical composition. It can be accomplished by means of the same mixing equipment as described above for the premixing step.

In one embodiment, the granulate is first mixed with filler and subsequently with glidant and lubricant. The duration of the mixing steps is not particularly restricted and may take any time from 0.1 to 100 minutes, preferably 1 to 30 minutes. For instance, in the above preferred embodiment, mixing with the filler may be carried out for a period of 3 to 25 minutes and preferably 5 to 20 minutes. The subsequent step of mixing with glidant and lubricant may be carried out for 0.5 to 5 minutes and preferably 1 to 3 minutes.

According to a particularly preferred embodiment, the granulate and lactose as a filler are mixed in a container mixer for 5 to 20 minutes. Afterwards colloidal silicone dioxide and sodium stearyl fumarate are added as glidant and lubricant, respectively, and mixed for additional 1 to 3 minutes in a container mixer.

### Capsulation

The final pharmaceutical composition may be filled into capsules, including hard- or soft-shell capsules. Suitable capsules may for instance be made of gelatin or HPMC. The size of the capsules is suitably selected having regard to the volume of the pharmaceutical composition.

For instance, size 0 capsules may be suitably used for the pharmaceutical compositions described in the examples below comprising 200 mg nilotinib and a total of 400 mg. In case of a lower strength of 150 mg nilotinib, size 1 capsules may be filled with an appropriate amount, for instance about 300 mg of the pharmaceutical compositions of the present invention as exemplified above.

Alternative to the filling of the pharmaceutical composition of the invention into capsule shells, it may be compressed into tablets or be filled into sachets or other suitable containers.

### Unit dosages

Unit dosages are not particularly restricted. It is however preferred to provide the pharmaceutical composition of the present invention in unit dosages of 200 mg, 150 mg or 50 mg (specified in terms of nilotinib weight, i.e. without taking the weight contribution of the salt and/or hydrate into account).

In case of unit dosages different from the unit dosages of the formulations described above and exemplified below, the formulations described above and exemplified below may be scaled in a linear manner up or down to the target dosage strength.

### Examples

Compositions having the formulations specified in the tables below are prepared using hot-melt granulation according to the following procedure:

### Procedure

Nilotinib hydrochloride dihydrate, lactose, crospovidone (in Examples 1, 2 and 4-6 only), Macrogol 8000 and polysorbate 20 (in Example 1 only) are premixed and added to a high shear mixer. The components are heated during mixing in a high shear mixer. The temperature of the high shear mixer heating coat is set to 70-90°C. The components in the mixer are mixed until the product reaches a temperature of 65 °C. This leads to melting of Macrogol 8000, which allows granules to form. The heating (increase of temperature until 65°C is reached) of the granulate varied between different samples and took time periods in the range of from 7 to almost 30 minutes. After the granulate is heated it is cooled while being mixed in the high shear mixer. It is cooled to a temperature of 45°C which is enough to solidify the meltable binder (Macrogol 8000). Afterwards the granulate is cooled in the fluid bed dryer to a temperature between 20 and 30 °C. After it is cooled, the granulate is sieved through a sieve with openings between 0.6 and 2.0 mm.

### Formulations

| | **Example 1** | | **Example 2** | | **Example 3** | |
|---|---|---|---|---|---|---|
| | mg | Percentage of capsule content | mg | Percentage of capsule content | mg | Percentage of capsule content |
| Composition of granulate | | | | | | |
| Nilotinib hydrochloride dihydrate | 227.37* | *56.84* | 227.37* | *56.84* | 227.37* | *56.84* |
| Lactose | 18.17 | *4.54* | 46.17 | *11.54* | 62.08 | *15.52* |
| Crospovidone | 15.91 | *3.98* | 15.91 | *3.98* | / | / |
| Macrogol 8000 powdered | 60.00 | *15.00* | 40.00 | *10.00* | 40.00 | *10.00* |
| Polysorbate 20 | 8.00 | *2* | / | / | / | / |

| Extragranular composition | | | | | | |
|---|---|---|---|---|---|---|
| Lactose | 60.45 | *15.11* | 60.45 | *15.11* | 60.45 | *15.11* |
| Colloidal silicon dioxide | 2.1 | *0.53* | 2.10 | *0.53* | 2.10 | *0.53* |
| Sodium stearyl fumarate | 8.00 | *2.00* | 8.00 | 2.00 | 8.00 | *2.00* |
| Sum of capsule content | 400.00 | *100.00* | 400.00 | *100.00* | 400.00 | *100.00* |
| Gelatine capsules size 0** | 96.00 | | 96.00 | | 96.00 | |

| | **Example 4** | | **Example 5** | | **Example 6** | |
|---|---|---|---|---|---|---|
| | mg | Percentage of capsule content | mg | Percentage of capsule content | mg | Percentage of capsule content |
| Composition of granulate | | | | | | |
| Nilotinib hydrochloride dihydrate | 227.37* | *56.84* | 227.37* | *56.84* | 227.37* | 54.14 |
| Lactose | 26.17 | *6.54* | 6.17 | *1.54* | 22.08 | *5.26* |
| Crospovidone | 15.91 | *3.98* | 15.91 | *3.98* | / | / |
| Macrogol 8000 powdered | 60.00 | *15.00* | 80.00 | *20.00* | 80.00 | *19.05* |

| Extragranular composition | | | | | | |
|---|---|---|---|---|---|---|
| Lactose | 60.45 | *15.11* | 60.45 | *15.11* | 79.940 | *19.03* |
| Colloidal silicon dioxide | 2.1 | *0.53* | 2.10 | *0.53* | 2.21 | *0.53* |
| Sodium stearyl fumarate | 8.00 | *2.00* | 8.00 | *2.00* | 8.40 | *2.00* |
| Sum of capsule content | 400.00 | *100.00* | 400.00 | *100.00* | 420 | *100.00* |
| Gelatine capsules size 0** | 96.00 | | 96.00 | | 96.00 | |

| | | | | | | |
|---|---|---|---|---|---|---|
| *The content of nilotinib hydrochloride dihydrate is equivalent to 200 mg of nilotinib. **For the 150 mg strength size 1 capsules are used. | | | | | | |

## Claims

1. Method of preparing a pharmaceutical composition comprising nilotinib or a pharmaceutically acceptable salt, hydrate or solvate thereof and a meltable binder, the method comprising a step wherein nilotinib or pharmaceutically acceptable salt, hydrate or solvate thereof, meltable binder and optionally one or more further excipients are subjected to hot melt granulation,
wherein nilotinib or the pharmaceutically acceptable salt, hydrate or solvate thereof is used in polymorphic form A,
wherein the meltable binder comprises a polyethylene glycol having an average molecular weight of from 5000 Da to 12,000 Da, and
wherein the process is carried out in the absence of water and/or organic liquids.

2. The method of claim 1, wherein the hot melt granulation step comprises the following individual steps:
a. Preparing a premix by mixing nilotinib or pharmaceutically acceptable salt, hydrate or solvate thereof, meltable binder and optionally one or more further excipients in a dry state nilotinib;
b. Subjecting the premix to high shear mixing conditions while increasing the temperature of the mixture from room temperature until the temperature of the mixture reaches a temperature above the melting point of the meltable binder
c. Cooling to a temperature of 35-45 °C while optionally continuing high shear mixing;
d. Further cooling to room temperature in a fluid bed dryer
e. Sieving.

3. The method of claim 1 or 2, wherein the granulate obtained in the hot melt granulation step is mixed with further excipients, said further excipients preferably comprising a filler, glidant and/or lubricant.

4. The method of anyone of claims 1 to 3, wherein nilotinib is used in the form of its hydrochloride salt.

5. The method of anyone of claims 1 to 4, wherein the meltable binder is or comprises macrogol 8000.

6. The method of anyone of claims 1 to 5, wherein lactose is used as a further excipient in the hot melt granulation step.

7. The method of anyone of claims 1 to 6, wherein crospovidone is used as a further excipient in the hot melt granulation step.

8. The method of anyone of claims 1 to 7, wherein a polyethylene glycol group-containing surfactant is used as a further excipient in the hot melt granulation step.

9. The method of anyone of claims 3 to 8, wherein lactose is used as a further excipient in the step of mixing the granulate with further excipients.

10. The method of anyone of claims 3 to 9, wherein colloidal silicon dioxide and/or sodium stearyl fumarate is used as a further excipient in the step of mixing the granulate with further excipients.

11. The method of anyone of claims 1 to 10, wherein the method comprises a further step of filling the pharmaceutical composition obtained according to anyone of claims 1 to 10 into a capsule shell.

12. The method of claim 11, wherein nilotinib is used in an amount such that each capsule contains an amount of nilotinib of 50 mg, 150 mg or 200 mg.

13. A pharmaceutical composition comprising nilotinib or a pharmaceutically acceptable salt, hydrate or solvate thereof and a meltable binder, which is obtainable by a method according to anyone of claims 1 to 12.

14. The pharmaceutical composition of claim 13, wherein the nilotinib contained in the pharmaceutical composition comprises 5 wt.%, on the basis of the entire weight of nilotinib being 100 wt.%, or less of polymorphic forms other than polymorphic form A.

15. A pharmaceutical composition comprising nilotinib or a pharmaceutically acceptable salt, hydrate or solvate thereof and a meltable binder as specified in claims 13 or 14 for use in the treatment of tumor diseases and especially Philadelphia-positive chronic myelogous leukaemia.
